# EUROPEAN PATENT SPECIFICATION

(11) **EP 3 400 918 B1**
(45) Date of publication and mention of the grant of the patent: **07.04.2021**
(21) Application number: 17170366.3
(22) Date of filing: 10.05.2017
(51) Int. Cl.: A61F 13/20, A61F 13/34

(54) **IMPROVED TAMPON WITHDRAWAL SYSTEM AND METHOD FOR THE FABRICATION THEREOF**
VERBESSERTES TAMPONENTNAHMESYSTEM SOWIE VERFAHREN ZUR HERSTELLUNG DAVON
SYSTÈME DE RETRAIT DE TAMPON AMÉLIORÉ ET SON PROCÉDÉ DE FABRICATION

(43) Date of publication of application: 14.11.2018
(73) Proprietor: Ontex BV, 9255 Buggenhout (BE); Ontex Group NV, 9320 Erembodegem (BE)
(72) Inventor: HEEGE, Rudolf, 56759 Kaiserses (DE); HEEGE, Thomas, 56761 Düngenheim (DE)
(74) Representative: Saurat, Thibault

(56) References cited:
- EP-A1- 1 108 407
- DE-A1- 2 926 130
- GB-A- 1 518 320
- GB-A- 1 521 028
- GB-A- 2 032 783

## Description

### Technical field

The invention pertains to the technical field of withdrawal systems for digital tampons, and methods for the fabrication thereof. More in particular, the invention describes digital tampons, comprising an essentially cylindrical absorbent body and a withdrawal string, in which the contact length of the withdrawal string and the absorbent body is enlarged to prevent telescoping and breaking of the withdrawal string. Furthermore, the invention relates to a method for fabrication of said tampons.

### Background

Tampons are well known in the art and are used for feminine hygiene. Also many tampon manufacturing methods and apparatuses have been disclosed in the prior art. Generally, a distinction is made between folded and rolled tampons. The former are commonly used with an applicator to reduce the chance of tears and other damage to the tampon. Most rolled tampons can be applied digitally, and comprise a rolled absorbent sheet and a withdrawal string for removal. The rolled absorbent sheet comprises an insertion end, a withdrawal end, and an essentially cylindrical body in between. The withdrawal string extends out of the withdrawal end. Sometimes rolled tampons also comprise a cover of a nonwoven, encapsulating the cylindrical body.

During the manufacturing process of rolled tampons, an absorbent sheet with mutually orthogonal width and length directions is provided. The withdrawal string is looped in the width direction around the absorbent sheet, and subsequently the absorbent sheet is rolled around a rolling axis parallel to the width direction. A commonly encountered problem with rolled tampons is telescoping. When force is applied to the withdrawal string, the force acts on a single inner winding of the rolled absorbent sheet (also called absorbent body), and said winding moves with respect to the other windings. Figure 1 of document WO 2015/104301 illustrates telescoping. For convenience we have included said figure in **FIG. 1****.** In addition, the withdrawal string sometimes breaks at the turning point of the loop around the rolled absorbent sheet, as most of the pulling force attacks at this point. Document WO 2015/104301 proposes a cover made of a nonwoven which completely covers at least the withdrawal end with the exception of a through-opening for the withdrawal means to prevent telescoping as well as the escape of fibers from the withdrawal end. The proposed solution in document WO 2015/104301 is illustrated in **FIGS. 2 and 3****.** In **FIG. 3****,** the cylindrical body comprises an absorbent body **1** and a cover made of a nonwoven **3** around the cylindrical body. In addition, a cover made of a nonwoven covers the withdrawal end **4** with the exception of a through-opening for the withdrawal string. However, the proposed solution in document WO 2015/104301 does not help to prevent breaking of the withdrawal string.

Document US 3,976,075 proposes an absorbent sheet having printed on at least one surface thereof a non-occlusive pattern of adhesive binder. The resulting rolled tampon has a reduced tendency for telescoping and for fibrous particles to break loose from the cylindrical body. In addition, the non-occlusive pattern of adhesive binder can help to prevent breaking of the withdrawal string at the turning point of the loop around the rolled absorbent sheet. The proposed solution in document US 3,976,075 is illustrated in **FIGS. 4 and 5****.**

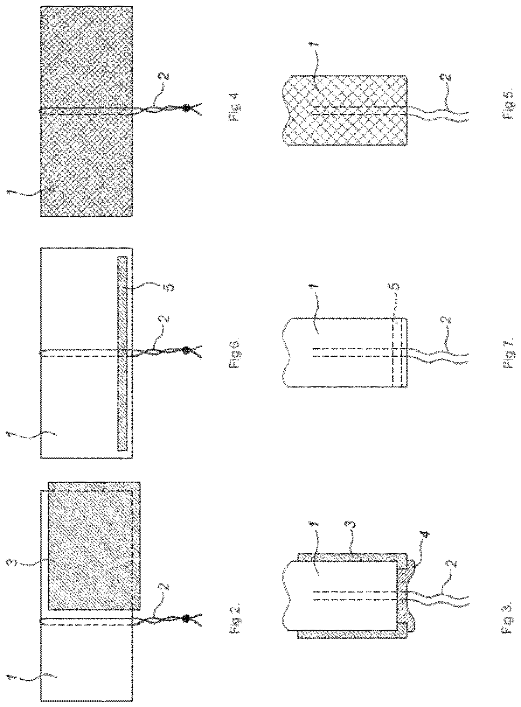

Document US 4,787,895 proposes to place a generally continuous line of adhesive along one side of the absorbent sheet parallel to the length direction. After rolling the sheet, the withdrawal string is connected adhesively to the cylindrical body, and the adhesive also interconnects the rolled absorbent sheet. This helps the tampon to resist telescoping during withdrawal and to prevent breaking of the withdrawal string at the turning point of the loop around the rolled absorbent sheet. The proposed solution in document US 4,787,895 is illustrated in **FIGS. 6 and 7****.** In **FIG. 6****,** the generally continuous line of adhesive **5** along one side of the absorbent sheet **1** parallel to the length direction connects the withdrawal string **2** adhesively to the absorbent sheet **1.** In **FIG. 7****,** the adhesive **5** also interconnects the rolled absorbent sheet **1.**

The abovementioned prior art has the common disadvantage that additional material and additional production steps are needed to achieve a greater resistance to telescoping and/or to prevent breaking of the withdrawal string at the turning point of the loop around the rolled absorbent sheet. The solution in document WO 2015/104301 requires extra nonwoven and the application of the extra nonwoven on the withdrawal end. The solution in document US 3,976,075 requires an extra non-occlusive pattern of adhesive binder and the application thereof on the absorbent sheet. The solution in document US 4,787,895 requires an extra generally continuous line of adhesive and the application thereof on the absorbent sheet.

DE2926130 is directed to a sanitary tampon with retrieval cord wound with spacing around strip of absorbent mat.

GB1521028 is directed to a menstrual tampon which comprises a fibrous mat with a withdrawal end and to methods of making such tampons.

EP1108407 is directed to digital tampons capable of radially expanding into a non-circular cross-sectional shape upon exposure to a wet environment, as weil as blanks and processes of making.

GB1518320 is directed to a menstrual tampon which comprises a fibrous mat with a withdrawal end and to methods of making such tampons.

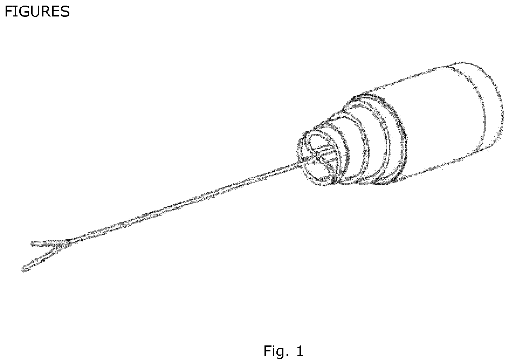

GB2032783 is directed to a tampon having a body of absorbing material with an extraction string extending from the body, the string being laid around the tampon e.g. helically (Figure 1) or lengthwise (Figure 2) prior to use.

Our solution circumvents this disadvantage.

### Summary of the invention

In a first aspect, the present invention pertains to a tampon according to claim 1. A path comprising a helical portion around the central axis, comprises a point on the path which does not lie on the axis, a longitudinal direction parallel to the central axis, a radial direction parallel to the shortest path connecting the point and the central axis, an orbital direction orthogonal to both the longitudinal and radial directions, and a tangent to the path through said point which comprises nonzero components along both the longitudinal and orbital directions.

The loop of the withdrawal string **2** around the absorbent sheet **1** in the solutions of the prior art in **FIGS. 2, 4, and 6** results in a minimal contact length between the withdrawal string **2** and the absorbent body **1** in **FIGS. 3, 5, and 7****,** respectively. When the string follows within the absorbent body a string path comprising a helical portion around the central axis, the contact length of the withdrawal string and the absorbent body is enlarged. As a result, the tampon has a greater resistance to telescoping as the pulling force is spread over multiple windings of the absorbent sheet, and as the helical portion of the string path snares the absorbent body. In addition, the shear forces of the withdrawal string and the absorbent body are spread over a larger contact length, and a larger force can be applied to the withdrawal string before the latter will break.

In a second aspect, the present invention pertains to a method for the fabrication of said tampon, according to claim 5.

The nonparallelity of the string application path with both the length and the width directions of the absorbent sheet results in an enlarged contact length of the string and the absorbent sheet. In the finished tampon, this corresponds to a string path comprising a helical portion around the central axis, and hence an enlarged contact length of the withdrawal string and the absorbent body. As a result, the tampon has a greater resistance to telescoping as the pulling force is spread over multiple windings of the absorbent sheet, and as the helical portion of the string path snares the absorbent body. In addition, the shear forces of the withdrawal string and the absorbent body are spread over a larger contact length, and a larger force can be applied to the withdrawal string before the latter will break.

In a preferred embodiment of the method, the method comprises the additional step in between steps c and d of folding the sheet and applied string, preferably around a folding axis essentially parallel to the width direction, more preferably around a folding axis essentially coinciding with the rolling axis.

In a preferred embodiment of the method, the method comprises the additional step before, during, or after step c and before step d of applying one or more nonwoven films comprising thermoplastic fibers fully or partially on to the absorbent sheet.

In a preferred embodiment of the method, in which the provided absorbent sheet extends significantly in the length direction, the method comprises the additional step before, during, or after step c and before step d of detaching a piece of the provided absorbent sheet, preferably by cutting.

In a preferred embodiment of the method, during step c the first portion of the string application path is essentially a straight line and the second portion of the string application path is essentially a straight line, whereby the lines make an angle with the width direction of preferably at least 5 degrees, more preferably at least 15 degrees, even more preferably at least 25 degrees, even more preferably at least 35 degrees, and even more preferably at least 45 degrees. In an even more preferred embodiment of the method, the two straight lines are essentially parallel.

### Description of figures

**Figure 1** is a schematic representation illustrating the effect of telescoping in rolled tampons.
**Figure 2** is a schematic representation illustrating an intermediate in the method to produce the rolled tampon described in document WO 2015/104301**.**
**Figure 3** is a schematic representation of a part of the rolled tampon described in document WO 2015/104301**.**
**Figure 4** is a schematic representation illustrating an intermediate in the method to produce the rolled tampon described in document US 3,976,075**.**
**Figure 5** is a schematic representation of a part of the rolled tampon described in document US 3,976,075**.**
**Figure 6** is a schematic representation illustrating an intermediate in the method to produce the rolled tampon described in document US 4,787,895**.**
**Figure 7** is a schematic representation of a part of the rolled tampon described in document US 4,787,895**.**
**Figures 8, 10****,** **12, 14****,** **16, and 18** are schematic representations of string application paths in methods to produce rolled tampons.
**Figures 9, 11****,** **13, 15****,** **17, and 19** are schematic representations of parts of rolled tampons comprising a withdrawal string with a string path comprising a helical portion around the central axis of the tampon.
**Figure 20** is a schematic representation of intermediate steps in the method to produce rolled tampons according to the present invention.
**Figure 21** is a schematic representation of a string application apparatus in two intermediate positions during string application according to the present invention.
**Figure 22** is a schematic representation of a string application apparatus in two intermediate positions during string application with a contact axis essentially parallel to the length direction and essentially perpendicular to the length direction of the absorbent sheet according to the present invention.
**Figure 23** is a schematic representation of a string application apparatus in two intermediate positions during string application with a contact axis essentially non-parallel to the width direction and non-parallel to the length direction of the absorbent sheet according to the present invention.

### Detailed description of the invention

The present invention concerns digital tampons with an improved withdrawal system, and methods for the fabrication thereof.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", "comprises" and "comprised of" as used herein are synonymous with "include", "including", "includes" or "contain", "containing", "contains" and are inclusive or open-ended terms that specify the presence of what follows e.g. component and do not exclude or preclude the presence of additional, non-recited components, features, elements, members, steps, known in the art or disclosed therein.

The term "nonwoven" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes.

The term "thermoplastic" is meant to describe a material that softens when exposed to heat and which substantially returns to its original condition when cooled to room temperature.

The term "string" is meant to describe a pliable long object with tensile strength. The term "string" as used herein can therefore be synonymous with "yarn", "thread", "twine", "cord", "lace", "line", or "rope". A string can comprise natural or synthetic fibers. Examples of natural fibers are manila hemp, hemp, feathers, linen, cotton, coir, jute, straw, sisal, silk, wool, and animal hair. Examples of synthetic fibers are polypropylene, nylon, polyester, polyethylene, cellulose, and acrylics. A string can also comprise mixtures of several fibers and/or comprise copolymer fibers. The fibers are spun and/or twisted and/or braided together into a larger and stronger form.

In a first aspect, the present invention pertains to a tampon comprising an absorbent body and a withdrawal string, whereby the absorbent body comprises an insertion end, a withdrawal end, and an essentially cylindrical body in between, whereby the cylindrical body further comprises a central axis, and whereby the withdrawal string extends out of the withdrawal end, characterized in that the withdrawal string follows within the absorbent body a string path comprising a helical portion around the central axis.

In the present invention, the withdrawal string follows within the absorbent body of the tampon a string path comprising a helical portion around the central axis. Herein, a helical portion around a central axis refers to a portion which does not lie on the axis and extends in a direction which has at least a longitudinal component and an orbital component, i.e. at any point of the helical portion, the tangent is not parallel to the central axis and does not lie in a plane perpendicular to the central axis. As a result, the helical portion curls around the central axis while also having a longitudinal extension. Optionally, the helical portion also has a radial component, meaning that the distance between the central axis and the helical portion is not the same for all points of the helical portion.

The loop of the withdrawal string 2 around the absorbent sheet 1 in the solutions of the prior art in **FIGS. 2, 4, and 6** results in a minimal contact length between the withdrawal string 2 and the absorbent body in **FIGS. 3, 5, and 7****,** respectively. When the string follows within the absorbent body a string path comprising a helical portion around the central axis, the contact length of the withdrawal string and the absorbent body is enlarged. As a result, the tampon has a greater resistance to telescoping as the pulling force is spread over multiple windings of the absorbent sheet, and as the helical portion of the string path snares the absorbent body. In addition, the shear forces of the withdrawal string and the absorbent body are spread over a larger contact length, and a larger force can be applied to the withdrawal string before the latter will break.

In a preferred embodiment of the present invention, the absorbent body of the tampon has a length measured along the central axis of preferably at least 2 cm and at most 15 cm, more preferably at least 3 cm and at most 11 cm, even more preferably at least 4 cm and at most 7 cm, such as 4 cm, 4.5 cm, 5 cm, 5.5 cm, 6 cm, 6.5 cm, 7 cm, or any value in between.

The contact ratio is the ratio of the contact length of the withdrawal string and the absorbent body to the length of the absorbent body measured along the central axis. In the current state-of-the-art, this contact ratio is about 2, as the withdrawal string follows a string path in the absorbent body substantially parallel to the central axis. According to the present invention, the contact ratio is at least 2.0075 and at most 4.7324, more preferably at least 2.8284 and at most 4.7324, such as 2.83, 2.93, 3.03, 3.13, 3.23, 3.33, 3.43, 3.53, 3.63, 3.73, 3.83, 3.93, 4.03, 4.13, 4.23, 4.33, 4.43, 4.53, 4.63, 4.73, or any value in between.

The telescoping resistance is a parameter which can be measured by clamping the cylindrical body of the tampon and by pulling with gradually increasing force the withdrawal cord in a direction parallel to the central axis of the tampon and away from the withdrawal end. Such measurement may be carried out by affixing (by use of clamp members or adhesive tape) the tampon to a first end and the withdrawal cord to a second end of a testcontroll II Zugprufmaschine (available at ZWICK GmbH & Co. KG) and apply a displacement of 1 cm/s moving said first and second ends in opposed directions. The force is recorded and the tampon is visually inspected during said displacement. The force at which the onset of telescoping occurs, as illustrated in **FIG. 1** and described above, is recorded and the latter being the telescoping resistance as referred to herein. In a preferred embodiment of the present invention, the telescoping resistance is preferably at least 5 N, more preferably at least 10 N, even more preferably at least 15 N, such as 15 N, 16 N, 17 N, 18 N, 19 N, 20 N, 22 N, 24 N, 26 N, 28 N, 30 N, 35 N, 40 N, 45 N, 50 N, or any value in between or above.

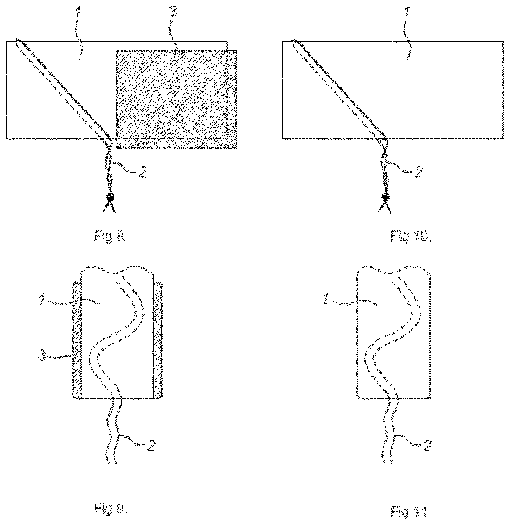

**FIG. 9** is a schematic representation of a part of a tampon, in which the withdrawal string **2** follows within the absorbent body **1** a string path comprising a helical portion around the central axis. Compared to **FIGS. 3, 5, and 7****,** the contact length of the withdrawal string **2** with the absorbent body **1** is larger in **FIG. 9****,** resulting in a greater resistance to telescoping, and a larger force can be applied to the withdrawal string.

In a preferred embodiment of the tampon, the absorbent body comprises a hydrophilic absorbent fiber web.

In a preferred embodiment of the tampon, the absorbent body comprises a nonwoven cover comprising thermoplastic fibers.

In a second aspect, the present invention pertains to a method for the fabrication of said tampon, according to claim 5.

The nonparallelity of the string application path with both the length and the width directions of the absorbent sheet results in an enlarged contact length of the string and the absorbent sheet. In the finished tampon, this corresponds to an enlarged contact length of the withdrawal string and the absorbent body. As a result, the tampon has a greater resistance to telescoping as the pulling force is spread over multiple windings of the absorbent sheet, and as the helical portion of the string path snares the absorbent body. In addition, the shear forces of the withdrawal string and the absorbent body are spread over a larger contact length, and a larger force can be applied to the withdrawal string before the latter will break.

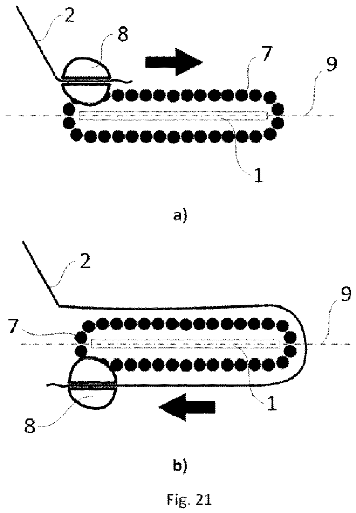

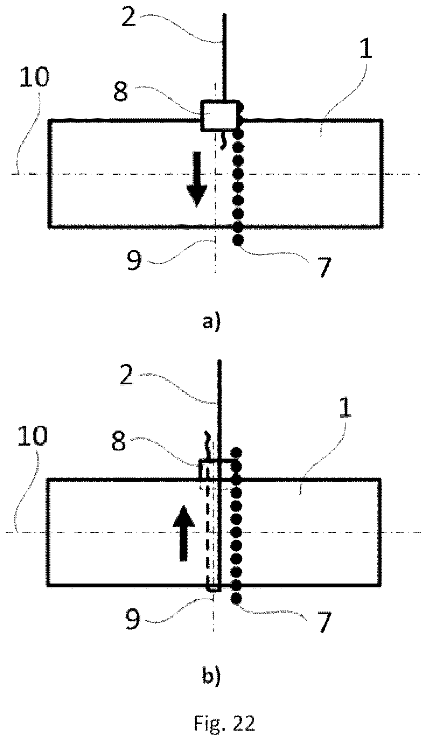

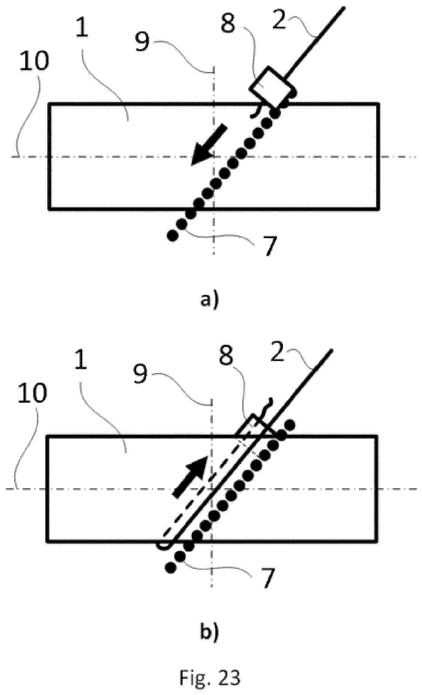

In an embodiment, the step of applying the string around the absorbent sheet is carried out by a string application apparatus and wherein at least a portion of said string application apparatus is arranged to contact the withdrawal string and/or absorbent sheet such that the string application path is substantially nonparallel to both the length direction and the width direction of the absorbent sheet, preferably wherein at least said portion of said string application apparatus comprising a contact axis that is at an angle substantially nonparallel to both the length axis **10** and the width axis **9** of the absorbent sheet, preferably said angle being from greater than 0° to less than 90°, more preferably between 15° and 75°, even more preferably between 25° and 65°, most preferably between 30° and 60°. As illustrated in **FIGS. 21****,** **22** **and** **23** typically said portion of the string application apparatus comprising a string contact element, which may comprise one or more movable clamp-shaped members **8,** extending substantially perpendicular to said contact axis, and one or more conveying means **7** such as a conveyor belt, extending substantially parallel to said contact axis, whereby said clamp-shaped members **8** are attached to said conveying means **7** and whereby said clamp-shaped members **8** hold said string **2** while moving along said conveying means **7** thereby leading said string **2** around said absorbent sheet **1** along said contact axis and whereby said string **2** is released by the clamp-shaped members 8 to contact said absorbent sheet **1** once it has been essentially entirely lead around said absorbent sheet **1.**

In a preferred embodiment of the method, the provided absorbent sheet has a width measured along the width direction of preferably at least 2 cm and at most 15 cm, more preferably at least 3 cm and at most 11 cm, even more preferably at least 4 cm and at most 7 cm, such as 4 cm, 4.5 cm, 5 cm, 5.5 cm, 6 cm, 6.5 cm, 7 cm, or any value in between.

In a preferred embodiment of the method, the method comprises the additional step in between steps c and d of folding the sheet and applied string, preferably around a folding axis essentially parallel to the width direction, more preferably around a folding axis essentially coinciding with the rolling axis.

In a preferred embodiment of the method, the method comprises the additional step before, during, or after step c and before step d of applying one or more nonwoven films comprising thermoplastic fibers fully or partially on to the absorbent sheet.

In a preferred embodiment of the method, in which the provided absorbent sheet extends significantly in the length direction, the method comprises the additional step before, during, or after step c and before step d of detaching a piece of the provided absorbent sheet, preferably by cutting.

According to the method of the invention, during step c the first portion of the string application path is essentially a straight line and the second portion of the string application path is essentially a straight line, whereby the lines make an angle with the width direction of preferably at least 5 degrees, more preferably at least 15 degrees, even more preferably at least 25 degrees, even more preferably at least 35 degrees, and even more preferably at least 45 degrees. According to the method of the invention, the two straight lines are essentially parallel. In a yet even more preferred embodiment of the method, the angle of the
two straight lines with the width direction is chosen to maximize the telescoping resistance of the finished tampon.

In a preferred embodiment of the method, the absorbent sheet comprises a hydrophilic absorbent fiber web.

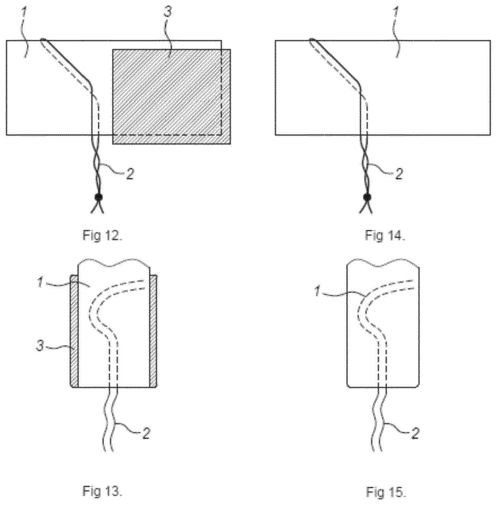

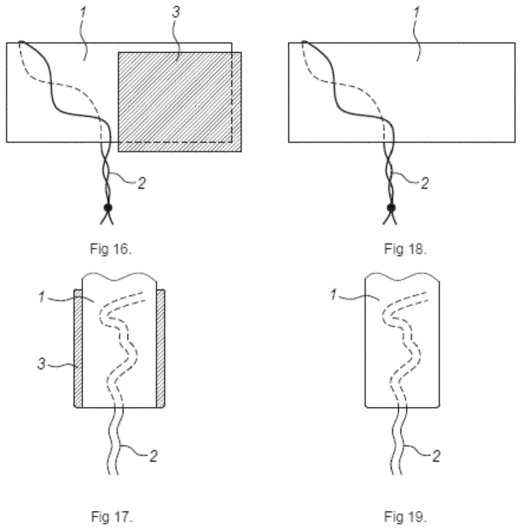

**FIGS. 10****,** **14****, and** **18** are schematic representations of an intermediate in a method to produce rolled tampons, in which the application path of the string 2 comprises a portion which is parallel to the absorbent sheet 1 and which is also significantly nonparallel to both the length and the width directions of the absorbent sheet **1.** **FIGS. 11****,** **15****, and** **19** show a part of the finished tampon based on the absorbent sheet **1** and applied string **2** in respectively **FIGS. 10****,** **14****, and** **18****.** In **FIG. 10****,** the portions of the string application paths in between the first and second length edges of the absorbent sheet are straight lines. In **FIG. 14****,** the portions of the string application paths in between the first and second length edges of the absorbent sheet are piecewise straight lines. In **FIG. 18****,** the portions of the string application paths in between the first and second length edges of the absorbent sheet have a continuously varying tangent.

**FIGS. 8****,** **12****, and** **16** are schematic representations of an intermediate in a method to produce rolled tampons, in which the application path of the string **2** comprises a portion which is parallel to the absorbent sheet **1** and which is also significantly nonparallel to both the length and the width directions of the absorbent sheet **1,** and in which a nonwoven film **3** comprising thermoplastic fibers has been applied on to the absorbent sheet **1.** **FIGS. 9****,** **13****, and** **17** show a part of the finished tampon based on the absorbent sheet **1,** applied string **2,** and nonwoven film **3** in respectively **FIGS. 8****,** **12****, and** **16****.** In **FIG. 8****,** the portions of the string application paths in between the first and second length edges of the absorbent sheet are straight lines. In **FIG. 12****,** the portions of the string application paths in between the first and second length edges of the absorbent sheet are piecewise straight lines. In **FIG. 16****,** the portions of the string application paths in between the first and second length edges of the absorbent sheet have a continuously varying tangent. **FIG. 12 to 19** are not in accordance with the method of the invention.

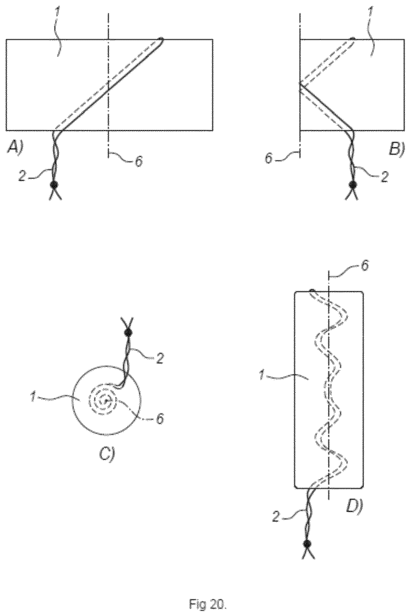

**FIG. 20** is schematic representation of intermediate steps in the method to produce rolled tampons according to the present invention. In **FIG. 20A** an absorbent sheet **1,** an applied string **2,** and a folding axis **6** are shown, whereby the string **2** follows a string path comprising a first straight line on the first side of the absorbent sheet **1** from the first length edge to the second length edge and a second straight line on the second side of the absorbent sheet **1** from the second length edge to the first length edge, whereby the two lines are essentially mutually parallel and essentially parallel to the absorbent sheet **1.** The sheet is subsequently folded around the folding axis **6,** resulting in the folded sheet **1** in **FIG 20B****.** The folding axis **6** also serves as rolling axis to produce a tampon preform. Due to the particular string application path in **FIG 20B****,** the string will follow inside the absorbent body a string path comprising two conical helices, with a common tip near the center of the essentially cylindrical body of the tampon preform. **FIG 20C** is a schematic representation of a projection of the string path inside the essentially cylindrical body of the tampon preform, as viewed from the withdrawal end. **FIG 20D** is a schematic representation of a projection of the string path inside the essentially cylindrical body of the tampon preform, as viewed from the side.

The invention is further described by the following non-limiting examples which further illustrate the invention, and are not intended to, nor should they be interpreted to, limit the scope of the invention.

### Examples

### Example 1: A preferred embodiment of the method and the finished tampon

A preferred embodiment of the method for the fabrication of a tampon, comprises the steps of
i. providing an absorbent sheet which comprises a hydrophilic absorbent fiber web, a first length edge, a second length edge essentially parallel to the first length edge, a length direction essentially parallel to the first and second length edges, a width direction parallel to the sheet and orthogonal to the length direction, a first side of the sheet, and a second side of the sheet,
ii. providing a nonwoven film comprising thermoplastic fibers,
iii. providing a string,
iv. applying the nonwoven film comprising thermoplastic fibers on to the absorbent sheet,
v. applying the string around the absorbent sheet, whereby the string follows a string path comprising a first straight line on the first side of the sheet from the first length edge to the second length edge and a second straight line on the second side of the sheet from the second length edge to the first length edge, whereby the two lines are essentially mutually parallel and essentially parallel to the absorbent sheet, and whereby the lines make an angle with the width direction of preferably at least 45 degrees,
vi. cutting the provided absorbent sheet, preferably along a cutting axis essentially parallel to the width direction, more preferably along a cutting axis essentially parallel to the width direction and not crossing the applied nonwoven film or the applied string,
vii. folding the sheet and applied string, preferably around a folding axis essentially parallel to the width direction, more preferably around a folding axis essentially parallel to the width direction and near the center of the detached piece of absorbent sheet,
viii. rolling up the sheet and applied string, preferably around a rolling axis essentially parallel to the width direction, more preferably around a rolling axis essentially coinciding with the folding axis, thereby obtaining a tampon preform, and
ix. finishing the tampon from the tampon preform.

### Example 2: Tampon string specifications

In an embodiment of the present invention, the string consists for 100% of organic cotton and has a tensile strength of 30 Newton.

In another embodiment of the present invention, the string consists for 100% of organic cotton and has a tensile strength of 70 Newton.

In yet another embodiment of the present invention, the string comprises 67% polyester and 33% cotton and has a tensile strength of 50 Newton.

## Claims

1. A tampon comprising an absorbent body and a withdrawal string (2), whereby the absorbent body comprises an insertion end, a withdrawal end (4), and an essentially cylindrical body in between, whereby the cylindrical body further comprises a central axis, and whereby the withdrawal string (2) extends out of the withdrawal end (4), **characterized in that** the withdrawal string (2) follows within the absorbent body a string path comprising a helical portion around the central axis, and wherein the withdrawal string has a contact ratio, defined as the ratio of the contact length of the withdrawal string and the absorbent body to the length of the absorbent body measured along the central axis, of at least 2.0075 and at most 4.7324.

2. A tampon according to claim 1, wherein the absorbent body comprises a hydrophilic absorbent fiber web.

3. A tampon according to any one of the previous claims, in which the absorbent body comprises a nonwoven cover comprising thermoplastic fibers.

4. A tampon according to any one of the previous claims, in which the telescoping resistance is at least 5 N, preferably at least 10 N, and more preferably at least 15 N.

5. Method for the fabrication of a tampon according to claim 1, comprising the steps of
a. providing an absorbent sheet (1) which comprises a first length edge, a second length edge essentially parallel to the first length edge, a length direction essentially parallel to the first and second length edges, a width direction parallel to the absorbent sheet and perpendicular to the length direction, a first side of the sheet, and a second side of the sheet,
b. providing a withdrawal string (2),
c. applying the withdrawal string (2) around the absorbent sheet (1), whereby the withdrawal string (2) follows a string path comprising a first portion on the first side of the absorbent sheet (1) from the first length edge to the second length edge and a second portion on the second side of the absorbent sheet (1) from the second length edge to the first length edge,
d. rolling up the absorbent sheet (1) and applied withdrawal string (2), preferably around a rolling axis essentially parallel to the width direction, thereby obtaining a tampon preform,
e. finishing the tampon from the tampon preform,
**characterized in that**, during step c the withdrawal string (2) follows a string application path comprising a portion which is substantially nonparallel to both the length direction and the width direction of the absorbent sheet (1), and **in that** during step c the first portion of the string application path is essentially a straight line and the second portion of the string application path is essentially a straight line, whereby the lines make an angle with the width direction, and wherein the straight line of the first portion and the straight line of the second portion are essentially parallel.

6. A method according to Claim 5, wherein step c comprises applying the string around the absorbent sheet (1) with a string application apparatus and wherein at least a portion of said string application apparatus is arranged to contact the withdrawal string (2) and/or absorbent sheet (1) such that the string application path is substantially nonparallel to both the length direction and the width direction of the absorbent sheet (2), preferably wherein at least said portion of said string application apparatus is positioned substantially nonparallel to both the length direction and the width direction of the absorbent sheet (2).

7. Method according to claim 5 or 6, comprising the additional step in between steps c and d of folding the absorbent sheet (1) and applied withdrawal string (2), preferably around a folding axis (6) essentially parallel to the width direction, more preferably around a folding axis (6) essentially coinciding with the rolling axis.

8. Method according to claims 5 to 7, comprising the additional step before, during, or after step c and before step d of applying one or more nonwoven films (3) comprising thermoplastic fibers fully or partially on to the absorbent sheet (1).

9. Method according to any of the claims 5 to 8, in which the absorbent sheet (1) is provided as an endless sheet in the length direction, and the method comprising the additional step before, during, or after step c and before step d of detaching a piece of the provided absorbent sheet (1), preferably by cutting the endless absorbent sheet (1) along the width direction.

10. Method according to any of the claims 5 to 9, in which the angle with the width direction is of at least 5 degrees, preferably at least 15 degrees, more preferably at least 25 degrees, even more preferably at least 35 degrees, and even more preferably at least 45 degrees.

11. Method according to any of the claims 5 to 10, in which the absorbent sheet (1) comprises a hydrophilic absorbent fiber web.

## Patentansprüche

1. Tampon, einen absorbierenden Hauptteil und ein Entfernungsband (2) umfassend, wobei der absorbierende Hauptteil ein Einführungsende, ein Entfernungsende (4) und dazwischen einen im Wesentlichen zylinderförmigen Hauptteil umfasst, wobei der zylinderförmige Hauptteil ferner eine Mittelachse umfasst und wobei sich das Entfernungsband (2) aus dem Entfernungsende (4) heraus erstreckt, **dadurch gekennzeichnet, dass** das Entfernungsband (2) in dem absorbierenden Hauptteil einem Bandweg folgt, der einen spiralförmigen Abschnitt um die Mittelachse herum umfasst, und wobei das Entfernungsband ein Kontaktverhältnis, das als das Verhältnis der Kontaktlänge des Entfernungsbandes und des absorbierenden Hauptteils zur Länge des absorbierenden Hauptteils, gemessen entlang der Mittelache, definiert ist, von mindestens 2,0075 und höchstens 4,7324 aufweist.

2. Tampon nach Anspruch 1, wobei der absorbierende Hauptteil eine hydrophile, absorbierende Faserbahn umfasst.

3. Tampon nach einem der vorhergehenden Ansprüche, wobei der absorbierende Hauptteil eine Vliesabdeckung umfasst, die Thermoplastfasern umfasst.

4. Tampon nach einem der vorhergehenden Ansprüche, wobei die Widerstandsfähigkeit gegen Zusammenschieben mindestens 5 N beträgt, vorzugsweise mindestens 10 N und noch bevorzugter mindestens 15 N.

5. Verfahren zur Herstellung eines Tampons nach Anspruch 1, die folgenden Schritte umfassend:
a. Bereitstellen einer absorbierenden Bahn (1), die einen ersten Längsrand, einen zweiten Längsrand, der im Wesentlichen parallel zum ersten Längsrand liegt, eine Längenrichtung, die im Wesentlichen parallel zum ersten und zum zweiten Längsrand liegt, ein Breiterichtung, die parallel zur absorbierenden Bahn und senkrecht zur Längenrichtung liegt, eine erste Seite der Bahn und eine zweite Seite der Bahn umfasst,
b. Bereitstellen eines Entfernungsbandes (2),
c. Anbringen des Entfernungsbandes (2) um die absorbierende Bahn (1) herum, wobei das Entfernungsband (2) einem Bandweg folgt, der einen ersten Abschnitt auf der ersten Seite der absorbierenden (1) Bahn von dem ersten Längsrand zum zweiten Längsrand und einen zweiten Abschnitt an der zweiten Seite der absorbierenden Bahn (1) von dem zweiten Längsrand zum ersten Längsrand umfasst,
d. Aufrollen der absorbierenden Bahn (1) und Anbringen des Entfernungsbandes (2), vorzugsweise um eine Rollachse herum, die im Wesentlichen parallel zur Breitenrichtung liegt, wodurch eine Tamponvorform erzielt wird,
e. Vollenden des Tampons aus der Tamponvorform,
**dadurch gekennzeichnet, dass** das Entfernungsband (2) während Schritt c einem Bandanbringungsweg folgt, der einen Abschnitt umfasst, der im Wesentlichen nicht parallel zu sowohl der Längenrichtung als auch der Breitenrichtung der absorbierenden Bahn (1) liegt, und dass während Schritt c der erste Abschnitt des Bandanbringungsweges im Wesentlichen eine gerade Linie ist und der zweite Abschnitt des Bandanbringungsweges im Wesentlichen eine gerade Linie ist und wobei die Linien mit der Breitenrichtung einen Winkel bilden und wobei die gerade Linie des ersten Abschnitts und die gerade Linie des zweiten Abschnitts im Wesentlichen parallel liegen.

6. Verfahren nach Anspruch 5, wobei Schritt c das Anbringen des Bandes um die absorbierende Bahn (1) herum mit einer Bandanbringungsvorrichtung umfasst und wobei mindestens ein Abschnitt der Bandanbringungsvorrichtung dafür angeordnet ist, in Kontakt mit dem Entfernungsband (2) und/oder der absorbierenden Bahn (1) zu gelangen, so dass der Bandanbringungsweg im Wesentlichen nicht parallel zu sowohl der Längenrichtung als auch der Breitenrichtung der absorbierenden Bahn (2) liegt, wobei vorzugsweise mindestens ein Abschnitt der Bandanbringungsvorrichtung im Wesentlichen nicht parallel zu sowohl der Längenrichtung als auch der Breitenrichtung der absorbierenden Bahn (2) liegt.

7. Verfahren nach Anspruch 5 oder 6, zwischen den Schritten c und d den zusätzlichen Schritt des Faltens der absorbierenden Bahn (1) und des angebrachten Entfernungsbandes (2), vorzugsweise um eine Faltachse (6), die im Wesentlichen parallel zur Breitenrichtung liegt, bevorzugter um eine Faltachse (6), die im Wesentlichen deckungsgleich mit der Rollachse ist, umfassend.

8. Verfahren nach einem der Ansprüche 5 bis 7, vor, während oder nach Schritt c und vor Schritt d den zusätzlichen Schritt des Anbringens eines oder mehrerer thermoplastfaserhaltiger Vliesfilme (3) vollständig oder teilweise an der absorbierenden Bahn (1) umfassend.

9. Verfahren nach einem der Ansprüche 5 bis 8, wobei die absorbierende Bahn (1) als eine in der Längenrichtung endlose Bahn bereitgestellt ist und das Verfahren vor, während oder nach Schritt c und vor Schritt d einen Schritt des Abtrennens eines Stücks der bereitgestellten absorbierenden Bahn (1), vorzugsweise durch Abschneiden der endlosen absorbierenden Bahn (1) entlang der Breitenrichtung, umfasst.

10. Verfahren nach einem der Ansprüche 5 bis 9, wobei der Winkel mit der Breitenrichtung mindestens 5 Grad beträgt, vorzugsweise mindestens 15 Grad, bevorzugter mindestens 25 Grad, noch bevorzugter mindestens 35 Grad und noch bevorzugter mindestens 45 Grad.

11. Verfahren nach einem der Ansprüche 5 bis 10, wobei die absorbierende Bahn (1) eine hydrophile absorbierende Faserbahn umfasst.

## Revendications

1. Tampon comprenant un corps absorbant et une ficelle de retrait (2), moyennant quoi le corps absorbant comprend une extrémité d'insertion, une extrémité de retrait (4), et un corps essentiellement cylindrique entre celles-ci, moyennant quoi le corps cylindrique comprend en outre un axe central, et moyennant quoi la ficelle de retrait (2) s'étend hors de l'extrémité de retrait (4), **caractérisé en ce que** la ficelle de retrait (2) suit, dans le corps absorbant, un trajet de ficelle comprenant une partie hélicoïdale autour de l'axe central, et dans lequel la ficelle de retrait possède un rapport de contact, défini comme le rapport entre la longueur de contact de la ficelle de retrait et du corps absorbant et la longueur du corps absorbant, mesurée le long de l'axe central, d'au moins 2,0075 et tout au plus de 4,7324.

2. Tampon selon la revendication 1, dans lequel le corps absorbant comprend une bande de fibres absorbantes hydrophiles.

3. Tampon selon l'une quelconque des revendications précédentes, dans lequel le corps absorbant comprend un revêtement non-tissé comprenant des fibres thermoplastiques.

4. Tampon selon l'une quelconque des revendications précédentes, dans lequel la résistance au télescopage est d'au moins 5 N, de préférence d'au moins 10 N, et de préférence d'au moins 15 N.

5. Procédé de fabrication d'un tampon selon la revendication 1, comprenant les étapes consistant à
a. prévoir une couche absorbante (1) qui comprend un bord d'une première longueur, un bord d'une seconde longueur essentiellement parallèle au bord d'une première longueur, une direction de longueur essentiellement parallèle aux bords d'une première et d'une seconde longueurs, une direction de largeur parallèle à la couche absorbante et perpendiculaire à la direction de longueur, un premier côté de la couche, et un second côté de la couche,
b. prévoir une ficelle de retrait (2),
c. appliquer la ficelle de retrait (2) autour de la couche absorbante (1), moyennant quoi la ficelle de retrait (2) suit un trajet de ficelle comprenant une première partie sur le premier côté de la couche absorbante (1), entre le bord d'une première longueur et le bord d'une seconde longueur, et une seconde partie sur le second côté de la couche absorbante (1), entre le bord d'une seconde longueur et le bord d'une première longueur,
d. enrouler la couche absorbante (1) et la ficelle de retrait appliquée (2), de préférence autour d'un axe d'enroulement essentiellement parallèle à la direction de largeur, afin d'obtenir une préforme de tampon,
e. finir le tampon à partir de la préforme de tampon, **caractérisé en ce que**, pendant l'étape c, la ficelle de retrait (2) suit un trajet d'application de ficelle comprenant une partie qui est sensiblement non-parallèle à la direction de longueur et à la direction de largeur de la couche absorbante (1), et **en ce que**, pendant l'étape c, la première partie du trajet d'application de ficelle est essentiellement une ligne droite, et la seconde partie du trajet d'application de ficelle est essentiellement une ligne droite, moyennant quoi les lignes forment un angle avec la direction de la largeur, et dans lequel la ligne droite de la première partie et la ligne droite de la seconde partie sont essentiellement parallèles.

6. Procédé selon la revendication 5, dans lequel l'étape c comprend l'application de la ficelle autour de la couche absorbante (1) avec un appareil d'application de ficelle, et dans lequel au moins une partie dudit appareil d'application de ficelle est prévue pour entrer en contact avec la ficelle de retrait (2) et/ou la couche absorbante (1) de sorte que le trajet d'application de ficelle soit sensiblement non-parallèle à la direction de longueur et à la direction de largeur de la couche absorbante (2), dans lequel, de préférence, au moins ladite partie dudit appareil d'application de ficelle est positionnée de manière sensiblement non-parallèle à la direction de longueur et à la direction de largeur de la couche absorbante (2).

7. Procédé selon la revendication 5 ou 6, comprenant l'étape supplémentaire entre les étapes c et d, qui consiste à plier la couche absorbante (1) et la ficelle de retrait appliquée (2), de préférence autour d'un axe de pliage (6) essentiellement parallèle à la direction de largeur, de préférence autour d'un axe de pliage (6) qui coïncide essentiellement avec l'axe d'enroulement.

8. Procédé selon les revendications 5 à 7, comprenant l'étape supplémentaire, avant, pendant ou après l'étape c et après l'étape d, qui consiste à appliquer un ou plusieurs film(s) non tissé(s) (3) comprenant des fibres thermoplastiques entièrement ou partiellement sur la couche absorbante (1).

9. Procédé selon l'une quelconque des revendications 5 à 8, dans lequel la couche absorbante (1) est prévue comme une couche sans fin dans la direction de longueur, et le procédé comprend l'étape supplémentaire avant, pendant ou après l'étape c et avant l'étape d qui consiste à détacher un morceau de la couche absorbante prévue (1), de préférence en coupant la couche absorbante sans fin (1) le long de la direction de largeur.

10. Procédé selon l'une quelconque des revendications 5 à 9, dans lequel l'angle avec la direction de largeur est d'au moins 5 degrés, de préférence d'au moins 15 degrés, de préférence d'au moins 25 degrés, et de préférence d'au moins 35 degrés, et de préférence d'au moins 45 degrés.

11. Procédé selon l'une quelconque des revendications 5 à 10, dans lequel la couche absorbante (1) comprend une bande de fibres absorbantes hydrophiles.
